# FASCICULE DE BREVET EUROPEEN

(11) **EP 2 087 122 B1**
(45) Date de publication et mention de la délivrance du brevet: **17.05.2017**
(21) Numéro de dépôt: 07821736.1
(22) Date de dépôt: 23.10.2007
(51) Int. Cl.: C12N 15/82, C12N 15/29, C12N 15/01, C12N 15/87

(54) **MAÏS PRESENTANT UNE BONNE DIGESTIBILITE ET UNE RESISTANCE AUX MALADIES**
MAIS MIT GUTER VERDAULICHKEIT UND KRANKHEITSRESISTENZ
MAIZE WITH GOOD DIGESTIBILITY AND DISEASE RESISTANT

(30) Priorité: 24.10.2006 FR 0609295
(43) Date de publication de la demande: 12.08.2009
(73) Titulaire: Biogemma, 75001 Paris (FR)
(72) Inventeur: MURIGNEUX, Alain, 63670 La Roche Blanche (FR); TATOUT, Christophe, 42110 Salt en Donzy (FR); MARTINANT, Jean-Pierre, 63910 Vertaizon (FR); GREZES-BESSET, Bruno, 31770 Colomiers (FR)
(74) Mandataire: Flesselles, Bruno F.G.
(86) Numéro de dépôt international: PCT/EP2007/061373
(87) Numéro de publication internationale: WO 2008/049849

(56) Documents cités:
- EP-A- 0 516 958
- EP-A- 1 000 543
- WO-A-99/10498
- WO-A-2006/035045
- FOFANA BOURLAYE ET AL: "Suppression of induced resistance in cucumber through disruption of the flavonoid pathway" PHYTOPATHOLOGY, vol. 95, no. 1, janvier 2005 (2005-01), pages 114-123, XP009081963 ISSN: 0031-949X
- LAMB C J ET AL: "Emerging strategies for enhancing crop resistance to microbial pathogens" BIO/TECHNOLOGY 1992 UNITED STATES, vol. 10, no. 11, 1992, pages 1436-1445, XP002032892 ISSN: 0733-222X
- BILY A C ET AL: "Dehydrodimers of ferulic acid in maize grain pericarp and aleurone: Resistance factors to Fusarium graminearum." PHYTOPATHOLOGY, vol. 93, no. 6, juin 2003 (2003-06), pages 712-719, XP001249375 ISSN: 0031-949X cité dans la demande
- BOUDET A M ET AL: "LIGIN GENETIC ENGINEERING" MOLECULAR BREEDING, KLUWER ACADEMIC PUBLISHERS, DORDRECHT, NL, vol. 2, janvier 1996 (1996-01), pages 25-39, XP002025844 ISSN: 1380-3743

## Description

La présente invention se rapporte au domaine de l'amélioration des plantes, en particulier de l'amélioration de la digestibilité du maïs, et de la tolérance du maïs aux pathogènes fongiques et notamment à la fusariose.

La présente invention concerne plus précisément la mise au point d'un allèle particulier du gène codant pour une isoforme de la cinnamate-4-hydroxylase ou C4H (EC 1.14.13.11) chez le maïs. La présence de cet allèle a pour conséquence une amélioration de la digestibilité et de la tolérance à la fusariose par rapport à un maïs isogénique ne possédant pas l'allèle. La quantité de lignine présente dans la plante est également diminuée.

La lignine est l'un des deux composants majoritaires de la paroi végétale avec la cellulose. La paroi végétale principalement constituée de cellulose, hémicellulose et de lignine offre à la cellule une barrière naturelle contre l'extérieur. De nombreuses études ont démontré que l'une des réponses aux stress biotiques (attaques pathogènes) ou abiotiques (sécheresse, vent...) consiste en un renforcement de la paroi végétale en particulier en une teneur supérieure en lignines. Par ailleurs, de nombreux secteurs agronomiques ou industriels voient leurs rendements directement liés à la teneur et/ou à la composition en lignine de la paroi, notamment l'industrie papetière, la production de combustibles (notamment la biomasse destinée aux biocarburants) ou la production d'ensilage.

Par exemple, on peut améliorer la qualité du maïs d'ensilage en diminuant la teneur en lignine ou en en modifiant la composition. L'ensilage de maïs est un aliment intéressant : le rendement au champ est relativement élevé, la récolte et le stockage sont aisés, les qualités nutritionnelles sont stables et peuvent aisément être complémentées en protéines par d'autres ensilages de fourrage ou par des tourteaux de soja. Une expérimentation menée par Emile (1995, Annales de zootechnie) démontre qu'alimenter du bétail avec un maïs plus digestible permet d'augmenter la production laitière jour et la prise de poids par rapport à une alimentation avec un maïs moins digestible. L'optimisation des qualités de l'ensilage de maïs consiste ainsi à augmenter l'énergie nette fournie par ce type d'alimentation en améliorant sa digestibilité et donc en diminuant la teneur en lignine.

Ainsi, la sélection ou l'obtention de plantes de maïs plus digestibles, notamment dont la voie de biosynthèse des lignines est modifiée est un des axes privilégiés d'amélioration du maïs. Il convient toutefois que les plantes sélectionnées possèdent de bons rendements et soient peu sensibles aux divers stress (mécanique, hydrique...).

Par ailleurs, les maïs sont soumis aux attaques de nombreux pathogènes, parmi lesquels les virus, bactéries, mais aussi les pathogènes fongiques, responsables de nombreuses maladies, et parfois de la présence de mycotoxines.

Ainsi, le maïs peut être attaqué par des champignons responsables de fusarioses (due aux *Fusarium,* dont *F. roseum, F. gramninearum, F. liseola, F. monoliforme),* de charbon (commun ou des inflorescences, du à *Ustilago zeae, Ustilago maydis),* d'anthracnose *(Colletotrichum graminicola),* de kabatiellose, d'helminthosporiose *(Helminthosporium turcicum),* de rouille (*Puccinia maydis*), de mildiou. D'une façon générale, les attaques fongiques sont responsables de dessèchement et/ou de pourriture des plantes, qui sont localisées différemment selon le pathogène.

Les champignons du genre *Fusarium* sont responsables de la fusariose. On peut citer les espèces *F. graminearum,* et *F. moniliforme* pathogènes du maïs et dont l'importance varie suivant les conditions climatiques et la précocité des variétés de maïs. On peut distinguer la fusariose de l'épi de la fusariose de la tige, les processus infectieux étant très différents. Certains agents pathogènes sont cependant communs aux deux types de fusariose.

La fusariose de l'épi qui entraîne une destruction des grains diminue le rendement des cultures de maïs. Les agents pathogènes en cause sont par ailleurs très dommageables car ils accumulent dans les grains détruits ou non différentes mycotoxines (Zéaralenone, Deoxynivalénol, Fumonisines), qui présentent des niveaux de toxicité variables suivant les espèces animales et sont difficiles à éliminer.

Les traitements fongicides sont difficiles d'utilisation et n'ont qu'un effet limité à l'encontre des *Fusarium.* La meilleure façon de combattre la fusariose de l'épi est l'utilisation de la résistance génétique. Peu d'hybrides possèdent actuellement une telle résistance, quand elle existe il s'agit d'un résistance partielle qui reste modérée.

La présente demande montre que l'inhibition du gène C4H et en particulier la présence de l'allèle D1938 du gène C4H de maïs permet d'obtenir un maïs à la fois plus digestible et présentant une meilleure tolérance aux maladies fongiques, et notamment à la fusariose.

Les maladies fongiques sont causées par des pathogènes fongiques tels que ceux décrits ci-avant.

Il est difficile de savoir comment modifier la voie de biosynthèse des lignines et de prévoir quelles seront les conséquences des modifications. En effet, la voie de biosynthèse des lignines reste une voie complexe, faisant intervenir un grand nombre de réactions enzymatiques (Dixon et al., 2001, Phytochemistry, 57(7), 1069-1084), et dont les éventuels mécanismes de compensation sont encore imparfaitement élucidés.

La lignine est considérée comme étant un polymère insoluble de 3 monomères d'alcools ou monolignols : l'alcool p-coumarylique (sous-unités H), l'alcool coniférylique (sous-unités G) et l'alcool sinapylique (sous-unités S), issus de la voie des phénylpropanoïdes (Neish, 1968, Constitution and Biosynthesis of lignin, eds New York, Springer Verlag 1-43). Chaque type de précurseur peut former une variété de liaisons avec d'autres et ainsi constituer la lignine. D'autres liaisons peuvent également s'établir avec d'autres composés pariétaux (polysaccharides et protéines) pour former un réseau complexe tridimensionnel.

Les principales étapes de fabrication de lignine sont l'hydroxylation, la O-méthylation des cycles aromatiques puis la conversion de la chaîne latérale carboxyl en une fonction alcool.

L'hypothèse actuelle de la voie de biosynthèse des monolignols considère que le réseau métabolique aboutissant à la formation des sous-unités S et G fait intervenir des réactions successives d'hydroxylations et de O-méthylations à différents niveaux de l'oxydation de la chaîne latérale. Les enzymes du réseau incluent :
- des O-méthyltransférases distinctes : la Caffeic acid 3-O Méthyltransférase (COMT) également appelée 5-hydroxyconiferyl aldéhyde O-méthyl transférase (AldOMT) et la Caffeoyl coenzyme A 3-O méthyltransférase (CCoAOMT)
- des hydroxycinnamate coenzyme A ligases (4CL)
- une ou des férulate 5-hydroxylase (F5H) à cytochrome P450
- et plusieurs isoformes de Cinnamoyl Co A réductase (CCR) et de Cinnamyl alcool dehydrogenase (CAD).

La propriétés de ces différentes enzymes ont fait l'objet de revues (Boudet et al, 1995 New Phytol. 129, 203-236; Dixon et al, 2001, précédemment cité; Whetten et al., 1998 Annu Rev. Plant Physiol Plant Mol Biol, 49, 585 -609, Li et al., 2000 J. Biol Chem, 275, 6537-6545).

Depuis plusieurs années, on a tenté de modifier les teneur et composition des plantes en lignines en surexprimant ou sous exprimant un ou plusieurs gènes de la voie de biosynthèse des lignines (Anterola et Lewis, 2002, Phytochemistry 61, 221-294). Notamment, les demandes de brevets (WO 9924561, EP0516958, WO9305160, WO9305159, WO9712982) exposent différentes stratégies imaginées. Cependant, la surexpression ou la sous expression d'une ou plusieurs enzymes ne donnent pas toujours des résultats constants et prévisibles.

La cinnamate 4-hydroxylase (C4H) existe au moins sous deux formes, en fonction des espèces considérées. La forme C4H-1 est impliquée dans la lignification et le métabolisme des phénylpropanoïdes, alors que le rôle de la C4H-2 est encore peu clair. Des expériences de dérégulation de la C4H-1 laissent entendre que ce gène est limitant dans la formation de lignines, la dérégulation résultant apparemment en une diminution progressive et une réduction quantitative (rapport S/G) de la lignine.

La demande de brevet EP 1000543 suggère une diminution de la quantité de cinnamate 4-hydroxylase (nommée CA4H dans cette demande) et de la Cinnamyl alcohol dehydrogenase (CAD) pour produire des aldéhydes utilisables pour améliorer la tolérance aux pathogènes fongiques. Cette demande précise toutefois que la réduction de la seule C4H est insuffisante pour obtenir ce résultat (paragraphe [0057], page 12). Cette demande ne décrit pas non plus de maïs présentant un gène C4H muté.

La demande de brevet WO 99/10498 décrit entre autres, la séquence du gène C4H de maïs. Cette demande ne décrit pas l'obtention des plantes de maïs possédant un gène de C4H muté, présentant une digestibilité améliorée, une tolérance accrue aux pathogènes fongiques et des propriétés agronomiques acceptables (rendement, résistance à la verse...). Cette demande ne décrit pas non plus la génération de plantes transgéniques sous-exprimant C4H et présentant une tolérance accrue aux pathogènes fongiques.

Il est ainsi difficile pour un homme du métier de prévoir les effets concrets d'une inhibition de la C4H seule ou d'une mutation spécifique de la C4H sur la digestibilité du maïs, sa tolérance aux pathogènes fongiques et la préservation d'un intérêt agronomique (rendement, résistance à la verse...).

La présente invention a pour objet de fournir à l'homme du métier un maïs qui possède effectivement une digestibilité améliorée et une tolérance accrue aux pathogènes fongiques, par la mise au point d'un allèle favorable de la C4H (appelé D1938), l'insertion d'un transposon ayant été effectuée après le nucléotide 2852 dans le gène représenté par SEQ ID N° 1, qui correspond à l'ADN génomique pour cette enzyme.

La séquence d'un ADNc (séquence 27 de WO 99/10498) est représentée par SEQ ID N° 2, la partie codante s'étendant des nucléotides 53 à 1555 pour cette séquence. Il est clair que ces séquences ne sont données qu'à titre d'exemples, et que l'homme du métier est à même d'identifier lui-même les séquences génomique et/ou d'ARNm de la C4H pour différentes variétés de maïs.

Le maïs selon l'invention présente une altération quantitative et/ou qualitative de la synthèse de la lignine.

Par « altération quantitative de la synthèse de lignine », on entend désigner une diminution de la quantité de lignine dans le maïs modifié selon l'invention par rapport à un maïs normal (témoin non modifié selon l'invention), évalué, par exemple par mesure de la lignine de Klason ou de la lignine obtenue par détergent acid (acid detergent lignin) selon des méthodes bien connues dans l'art (voir par exemple Jung et al., J Agric Food Chem. 1999 May;47(5):2005-8 ; Jung et al, J Dairy Sci. 1997 Aug;80(8):1622-8).

Par « altération qualitative de la synthèse de lignine », on entend désigner une modification dans la composition de la lignine de la plante modifiée selon l'invention par rapport à une plante témoin (non modifiée selon l'invention), par exemple une variation du rapport des sous unités S/G, ou une modification de la qualité d'acide féruliquc. Les méthodes d'analyse qualitative de la lignine sont également connues dans l'art. On peut notamment citer la RMN

Des graines possédant l'allèle D1938 ont été déposées au NCIMB Limited, Ferguson Building, Craibstone Estate, Bucksburn, Aberdeen, Scotland, AB21 9YA, UK, le 15 octobre 2007, selon les dispositions du Traité de Budapest, sous le numéro NCIMB 41507.

L'invention concerne notamment une plante de maïs, ou un grain de maïs possédant ledit allèle D1938. L'invention se rapporte également à un maïs ou un grain de maïs possédant à la fois l'allèle D1938 et un allèle du gène CCR1, appelé Δ3318, ledit allèle Δ3318 étant présent dans un échantillon représentatif de graines déposées au NCIMB sous le numéro NCIMB 41236 le 23 juillet 2004, selon les dispositions du traité de Budapest. Cet allèle Δ3318 est décrit dans la demande WO 2006/035045, dont l'enseignement est utilisable dans l'invention.

Cette plante montre une perturbation / altération de l'expression du gène C4H et/ou de l'enzyme codée par ce gène de telle sorte que la teneur en lignine est diminuée d'au moins 5 %, et la fraction digestible des parois est augmentée de 5 %, par rapport à des plantes quasi-isogéniques ne présentant pas cet allèle menant à une telle inhibition de l'activité du gène C4H.

Ainsi, la présente invention se rapporte notamment à une plante de maïs présentant une augmentation de la digestibilité (mesurée par NIRS) d'au moins 5 %, de préférence d'au moins 10 %, de façon plus préférée d'au moins 15 %.

De façon préférée, le maïs selon l'invention est un maïs « élite ». L'homme du métier connaît bien la définition d'un maïs élite. Par maïs élite, on entend un maïs destiné à générer des hybrides destinés à être commercialisés par croisement avec un autre maïs élite. Un maïs élite est défini comme tel en relation avec le territoire envisagé pour la commercialisation, ainsi que le(s) caractère(s) agronomique(s) recherché(s) pour la descendance hybride. Il s'agit notamment d'un maïs pouvant être inscrit dans un catalogue de référence.

Ainsi, selon que la descendance est destinée à l'alimentation humaine ou animale, on recherchera par exemple respectivement un rendement en grains ou un rendement à l'hectare et une bonne digestibilité, lorsque l'on évalue la nature « élite du maïs ».

Afin de déterminer le caractère élite d'un maïs, on compare des hybrides obtenus à partir de celui-ci aux hybrides commerciaux de référence (vendus pour le même objectif dans la même région), par des essais en champs, par relevé et mesures de caractères agronomiques appropriés à l'objectif recherché. Un maïs est défini comme élite si les résultats obtenus paramètres étudiés pour un hybrid obtenu par croisement dudit maïs sont supérieurs à 90 % aux résultats relevés pour les mêmes paramètres des hybrides de référence. Dans le cadre de la présente invention, on étudie notamment le caractère de digestibilité (digestibilité mesurée par NIRS (near infrared spectroscopy), par exemple).

La spectroscopie par proche infrarouge (NIRS) est la mesure de la longueur d'onde et l'intensité d'absorption de la lumière proche infrarouge par un échantillon, dans les domaines 800 nm - 2.5 ium (12,500 - 4000 cm-1). Cette spectroscopie est typiquement utilisée pour des mesures quantitatives de groupes fonctionnels organiques, en particulier O-H, N-H et C=O. Cette méthode est couramment utilisée dans l'analyse de la digestibilité d'échantillons.

Ainsi, un maïs élite est un maïs rassemblant le maximum de caractéristiques agronomiques nécessaires pour une pénétration économique du marché visé. Le marché du maïs étant aujourd'hui un marché d'hybrides, l'évaluation du caractère élite d'un maïs s'effectue également par l'aptitude dudit maïs à la combinaison/production d'hybrides.

Ainsi, la demande décrit un maïs élite destiné à la commercialisation d'hybrides pour l'alimentation animale et l'ensilage, présentant l'allèle D1938. Ce maïs élite est donc homozygote pour l'allèle D1938.

Dans un autre mode de réalisation, la demande décrit à un maïs hybride obtenu par croisement de deux lignées parentes homozygotes, ledit maïs hybride présentant un allèle D1938. Ce maïs hybride peut être homozygote (si chaque parent homozygote présente l'allèle D1938) ou hétérozygote pour l'allèle D1938.

L'invention permet également d'obtenir un maïs ou un grain de maïs, contenant un ou plusieurs transgènes en plus de l'allèle D1938. On peut citer des transgènes conférant une stérilité mâle, une fertilité mâle, la résistance à un herbicide (notamment le glyphosate, le glufosinate, l'imidazolinone, la sulfonylurée, la L- phosphinotricine, la triazine, le benzonitrile), la résistance aux insectes (notamment un transgène codant pour une toxine de *Bacillus thuringiensis*), la tolérance au stress hydrique. Ces maïs peuvent être obtenus en croisant un maïs contenant l'allèle D1938 avec un maïs contenant ledit transgène. La mise en oeuvre de rétrocroisements suivi d'une autofécondation permet d'obtenir un maïs élite homozygote pour l'allèle D1938 et le trangène. Toutefois, un maïs hybride contenant à la fois l'allèle D1938 et le transgène peut également être obtenu par la mise en oeuvre de l'invention.

La présente invention fournit également à l'homme de métier les moyens permettant de sélectionner les plantes de maïs possédant ces caractéristiques de digestibilité améliorée et de tolérance aux pathogènes fongiques. Il suffit en effet d'effectuer une PCR, ou un Southern Blot (hybridation de l'ADN génomique sur membrane) pour suivre la présence de l'insertion dans le dernier exon du gène codant pour la C4H. L'homme du métier peut aisément déterminer les amorces et sondes permettant d'identifier la présence de l'allèle D1938. L'invention permet ainsi également à une méthode de suivi de l'allèle D1938, par des techniques de biologie moléculaires, et notamment via la PCR en utilisant les amorces mentionnées dans les exemples.

L'invention permet également d'obtenir des plantes de maïs possédant une digestibilité améliorée et une tolérance aux pathogènes fongiques grâce à l'allèle D1938.

L'invention permet de mettre en oeuvre une méthode pour obtenir une lignée de maïs présentant de meilleures digestibilité et tolérance aux maladies fongiques, comprenant l'étape d'introgression de l'allèle D1938, dans une lignée de référence, présentant un caractère agronomique de qualité. L'introgression du caractère est notamment réalisée par sélection, selon les méthodes connues dans l'art (croisements et auto-fécondation). On sélectionne notamment les plantes à l'aide de marqueurs moléculaires.

Le principe en est rappelé ci-dessous :
On réalise une série de rétrocroisements (back-cross) entre la lignée élite et la lignée portant l'allèle D1938 (site unique sur le chromosome 5L).

Au cours des rétrocroisements, on peut sélectionner les individus porteurs de l'allèle D1938, et ayant recombiné le plus petit fragment de la lignée donneuse autour de cet allèle. En effet, grâce aux marqueurs moléculaires, on sélectionne les individus ayant pour les marqueurs proches du gène, le génotype de la lignée élite.

De plus, il est également possible d'accélérer le retour vers le parent élite grâce aux marqueurs moléculaires répartis sur l'ensemble du génome. A chaque rétrocroisement, seront choisis les individus ayant le plus de fragments issus du parent élite récurrent.

Avec une bonne mise en oeuvre, dès la quatrième génération, on peut obtenir une lignée quasi isogénique de la lignée élite, c'est-à-dire identique à la lignée élite de départ, mais ayant intégré le locus portant l'allèle D1938.

Ainsi, dans un mode de réalisation préféré, ladite méthode comprend les étapes consistant à :
a) croiser une première lignée de maïs présentant l'allèle D1938 avec une seconde lignée de maïs ne présentant pas ledit allèle,
b) effectuer un génotypage de la descendance obtenue et sélectionner les descendants présentant l'allèle D1938 ayant le meilleur génome ratio pour ce qui est de ladite seconde lignée,
c) effectuer un rétrocroisement desdits descendants avec ladite seconde lignée de maïs,
d) répéter si nécessaire les étapes b) et c) jusqu'à obtenir une lignée isogénique de ladite seconde lignée de maïs, présentant l'allèle D1938,
e) de façon optionnelle, effectuer une auto-fécondation afin d'obtenir une plante homozygote pour l'allèle D1938.

Le génotypage de l'étape b) est effectué préférentiellement en utilisant des marqueurs moléculaires (marqueurs microsatellites par exemple), permettant de définir la part de chacun des deux parents dans la descendance. On sélectionne également, dans la descendance, les maïs qui possèdent le caractère génétique approprié pour ce qui est de l'allèle D1938, de façon classique, par les méthodes de biologie moléculaire (telles que PCR ou Southern Blot).

De manière surprenante, il a été montré que la répétition des rétrocroisements entre les lignées sélectionnées à l'étape b) et le second maïs permet d'obtenir l'apparition d'un phénotype bien plus marqué au sein dudit second maïs.

Ce résultat est tout à fait surprenant car on aurait pu s'attendre à observer une amélioration de la digestibilité dès le premier croisement du maïs présentant l'allèle D1938 avec le second maïs.

L'invention permet également la mise en oeuvre d'une méthode dans laquelle on introgresse également l'allèle A3318 dans le maïs dans lequel on introgresse l'allèle D1938. L'introgression de A3318 est notamment effectuée selon les enseignements de WO 2006/035045, en utilisant notamment les amorces décrites dans les exemples de cette demande.

Par ailleurs, les résultats agronomiques observés après de multiples rétrocroisements (5 rétrocroisements et 2 auto-fécondations) ne montrent pas de différence entre les lignées isogéniques présentant la mutation et les plantes témoins.

De façon plus large, l'invention se rapporte également à une méthode pour augmenter la tolérance à un pathogène fongique chez le maïs comprenant une étape consistant à altérer qualitativement et/ou quantitativement la synthèse de lignines, par l'inhibition totale ou partielle de l'expression du gène codant pour la cinnamate 4-hydroxylase (C4H), la tolérance audit pathogène fongique étant augmentée par rapport à un maïs non altéré. La séquence d'un allèle du gène C4H est SEQ ID N°1, l'ADNc étant représenté par SEQ ID N° 2. Cette inhibition est réalisée par insertion d'un transposon tel que décrit plus haut pour l'allèle D1938.

Il convient de noter que les séquences fournies dans la liste de séquences ne doivent être considérées qu'en tant qu'illustrations de cet allèle. Il est clair que l'homme du métier, en utilisant ces séquences, est capable d'isoler ce gène C4H pour d'autres variétés de maïs, notamment en isolant, dans le génome d'une autre variété, l'allèle considéré par PCR, ou Southern Blot, puis en le séquençant.

La demande décrit aussi d'autres modes de réalisation de l'inhibition du gène C4H qui peut être obtenue par tout moyen connu dans l'art. Ainsi, on peut procéder à la mutation des gènes par insertion d'un élément transposable ou d'un ADN de transfert (T-DNA). On peut aussi effectuer une
mutagenèse physique ou chimique, notamment par l'utilisation d'EMS, de rayons X ou d'ultraviolets.

Les plantes ainsi mutées sont criblées par exemple par PCR, en utilisant des amorces situées dans le gène cible. Toutefois, on peut aussi utiliser d'autres méthodes de criblage, comme les Southern Blots ou un criblage via la méthode AIMS décrite dans WO 99/27085 (pour détecter les insertions), en utilisant des sondes spécifiques des gènes cibles, ou les méthodes de détection de mutations ponctuelles ou de petites insertions/délétions utilisant des endonucléases particulières (Cel I, Endo I) telles que décrites dans WO 2006/010646.

Dans un autre mode de réalisation, l'inhibition est obtenue par transformation de la plante avec un vecteur contenant un construit sens ou anti-sens du gène cible. Ces deux méthodes sont connues pour permettre, dans certaines conditions, l'inhibition du gène cible. On utilise également la méthode de l'interférence d'ARN (RNAi) qui est particulièrement efficace pour l'extinction de gènes dans les plantes. Cette méthode est bien connue de l'homme du métier et consiste en la transformation de la plante avec un construit produisant, après transcription, un duplex double-brin d'ARN, dont l'un des brins est complémentaire à l'ARNm du gène cible.

Enfin, l'invention se rapporte à l'utilisation d'un maïs selon l'invention, pour la préparation d'une composition destinée à l'alimentation animale, à une méthode pour la préparation d'une composition destinée à l'alimentation animale comprenant l'ensilage d'un maïs selon l'invention, ainsi qu'à la composition destinée à l'alimentation animale, ainsi obtenue. En particulier, ledit maïs est particulièrement utile pour l'alimentation du bétail.

### DESCRIPTION DES FIGURES

Figure 1 : représentation schématique de l'insertion dans le second intron du gène C4H. Les blocs gris correspondent aux exons, les traits correspondent aux introns. La position de deux amorces C4HH14intsp7 et C4H-3_comp permettant un suivi de la présence de l'insertion est représentée.
Figure 2 : illustration d'une méthode permettant le suivi de l'introgression de l'allèle D1938. Résultats d'amplification sur gel d'agarose.
Figure 3 : résultats NIR de l'introgression de l'allèle D1938 sur la quantité de lignine. Plantes mutantes : possédant l'allèle D1938; plantes contrôles : plantes ne possédant pas cet allèle.
Figure 4 : mesure de l'effet de l'allèle D1938 sur la fraction digestible des parois. Plantes mutantes : possédant l'allèle D1938; plantes contrôles : plantes ne possédant pas cet allèle.

### EXEMPLES

### Exemple 1. Description d'un maïs présentant une altération dans le gène de C4H

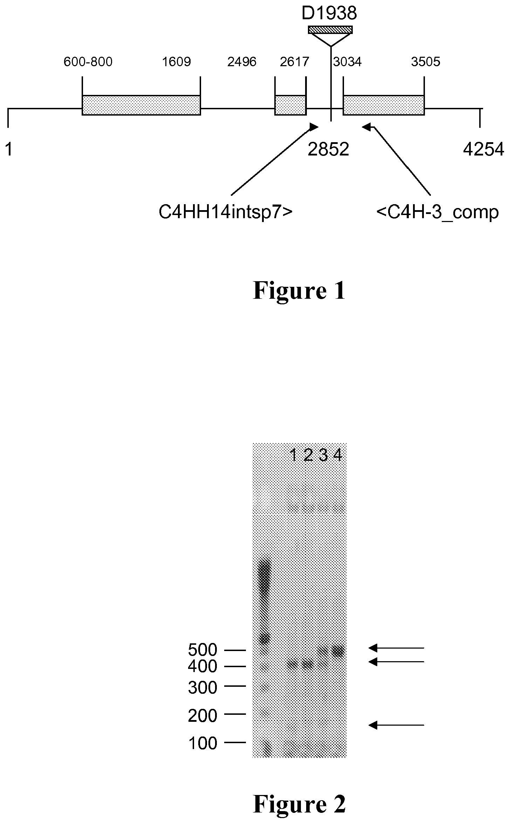

Une lignée de maïs présentant une insertion d'un élément transposable à la position 2852 de la séquence de référence SEQ ID N°1 est isolée (figure 1). L'allèle ainsi obtenu est appelé D1938.

Bien que présente dans un intron, on suppose que cette insertion a pour conséquence de déréguler la transcription et/ou la traduction du gène C4H, ou la stabilité de l'ARNm de C4H, menant à une diminution de l'activité de l'enzyme en présence de l'allèle D1938, ainsi qu'attesté par les résultats biochimiques de composition et teneur en lignine (exemple 2).

Afin de déterminer si l'insertion est sous forme homozygote ou hétérozygote, un couple d'amorces a été défini : amorce sens C4HH14intsp7 de séquence SEQ ID N° 3 : CACGTCTTAATCAAGTCTCCG et une amorce antisens C4H-3_comp de séquence SEQ ID N° 4 : GTTCATGTGGGGGACCAGCAGC.

En plus de ces deux amorces l'amorce OMuA (SEQ ID N°5) : CTTCGTCCATAATGGCAATTATCTC spécifique de l'élément transposable endogène est utilisée. Cette amorce est dirigée vers l'extrémité du transposon.

Ces trois amorces peuvent être utilisées simultanément dans une expérience d'amplification PCR à partir d'ADN génomique (Température d'hybridation = 58°C). Le dépôt sur gel des produits d'amplification révèle :
- l'obtention d'une seule bande d'environ 530 pb de long pour des plantes dites sauvages à ce locus (c'est à dire n'ayant pas la mutation) ;
- l'obtention de deux bandes d'environ 150pb et 420 pb pour des plantes homozygotes mutantes, correspondant aux amplifications obtenues avec les amorces présentes dans le gène et dans le transposon (du fait de l'insertion, l'amplification avec les amorces C4HH14intsp7 et C4H-3_comp est impossible car trop longue) ;
- ou l'obtention de l'ensemble des trois bandes pour des plantes hétérozygotes.

Ces résultats sont illustrés sur la Figure 2.
Le puits le plus à gauche sur le gel contient le marqueur de taille : la bande la plus basse correspond à 100 pb et il y a 100 pb entre chaque bande
Le puits 1 et 2 correspondent à des individus homozygotes mutants pour D1938.
Le puits 4 correspond à un individu sauvage ne portant pas l'allèle D1938.
Le puits 3 correspond à un individu hétérozygote.

### Exemple 2. Analyse phénotypique du mutant D1938 pour le caractère digestibilité

Afin d'étudier plus précisément l'effet de l'insertion observée dans le gène C4H dans un maïs élite, on effectue des back-cross (rétrocroisements) successifs avec une lignée élite de maïs.

Cette méthode permet très rapidement d'obtenir des lignées quasi isogéniques ne différant que par le locus portant l'allèle modifié, les descendants étant testés pour posséder un ratio génome le plus proche possible de celui du parent élite tout en ayant l'allèle que l'on désire introgresser. Ces tests sont assistés par marqueurs moléculaires (techniques bien connues, microsatellites, AFLP...). Pour essayer d'apprécier l'effet de l'insertion au plus tôt (obtention de plantes homozygotes), des autofécondations sont réalisées aux différents stades intermédiaires de back cross.

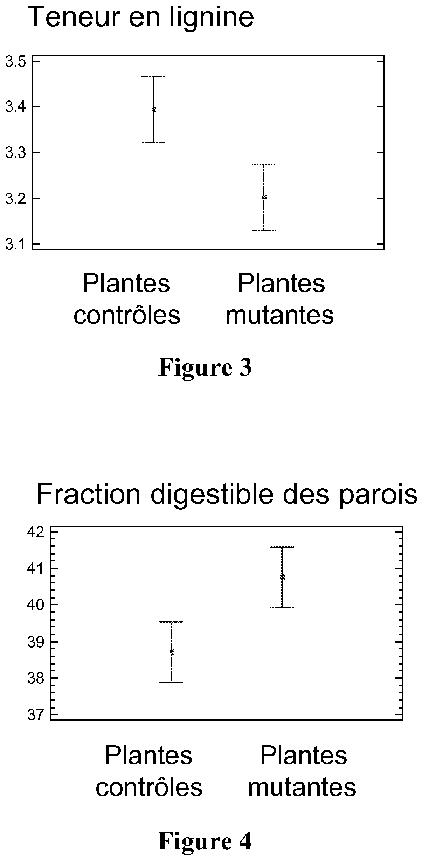

Les figures 3 et 4 présentent des résultats NIR et de digestibilité de paroi obtenus sur des plantes BC5S2 (5 rétrocroisements et 2 auto-fécondations).

Ceci montre que l'introgression de l'allèle D1938 permet d'obtenir une baisse de la quantité de lignine (Acid Detergent Lignin) après isolement des parois (Neutral Detergent Fibers), selon les méthodes connues de l'homme du métier.

Les différences observées entre les plantes contrôles et mutantes sont statistiquement significatives.

Les résultats sont résumés dans le tableau suivant :

| | Plantes contrôles (moyenne) | Plantes mutantes (moyennes) |
|---|---|---|
| Teneur en lignine = ADL | 3.39 | 3.20 |
| Fraction digestible des parois | 38.71 | 40.75 |

### Exemple 3. Analyse phénotypique du mutant D1938 pour le caractère de résistance aux maladies fongiques

On dispose d'une plante mutante homozygote et d'un contrôle homozygote sauvage pour chaque événement d'insertion. Etant donné les stades d'introgression de la mutation, on peut considérer que le mutant et le sauvage ne diffèrent que par la présence ou non de la mutation. L'expérimentation est réalisée selon le protocole suivant :
- 2 lieux
- 3 répétitions par lieu
- Inoculation artificielle de Fusarium monoliforme
- Notation des symptômes observés sur épis (note de 1 à 7). Il s'agit d'une notation visuelle d'intensité d'attaque sur épis : la note d'intensité d'attaque est calculée à partir du pourcentage de la surface de l'épi attaqué par le pathogène (Reid et alAgriculture and Agri-Food Canada, Ottawa, Ont. Technical Bulletin 1996-5E. 40 pp). Les notes correspondent à : 1 = 0% attaqué ; 2 = 1-3% ; 3 = 4-10% ; 4 = 11-25% ; 5 = 26-50% ; 6 = 51-75% ; 7 = 76-100%.

Dans les exemples ci-dessous, les répétitions « lieux » ont été converties en « répétitions simples » de façon à réaliser une analyse statistique simple.

L'analyse statistique a été réalisée sur chaque mutant de façon à savoir s'il existe une différence entre mutant (M) et sauvage ou témoin (S).

Les résultats démontrent que la présence de l'allèle D1938 du gène C4H augmente la tolérance à une infection par *Fusarium monoliforme.*

| TYPE | Effectif | Moyennes | Ecarts-types |
|---|---|---|---|
| **Mutant** | **72** | **2,9163** | **0,124743** |
| **Contrôle** | **81** | **3,8593** | **0,117351** |

| Contraste | Différence | | +/- limites |
|---|---|---|---|
| | ***-0,942997** | | **0,338582** |

| | | | |
|---|---|---|---|
| * **différence statistiquement significative.** | | | |

Ces résultats démontrent que l'altération de l'expression du gène C4H permet de diminuer les symptômes observés après infection par *Fusarium,* et donc d'améliorer la tolérance des plantes à ce pathogène.

### Exemple 4 : Réalisation de plantes transgéniques portant une cassette d'expression permettant l'inhibition d'un gène C4H.

### a- Transformation par Agrobacterium tumefaciens de plantes de maïs par un construit antisens C4H.

La transformation du maïs par *Agrobacterium tumefaciens,* est réalisée à l'aide d'un vecteur sous la forme d'un plasmide d'environ 50 kb obtenu par recombinaison dans *Agrobacterium* entre un vecteur pBIOS et un vecteur superbinaire (pSBl). Ce vecteur comporte notamment : une région d'origine de réplication plasmidique Col El, nécessaire au maintien et à la multiplication du plasmide dans *Escherichia coli,* non fonctionnelle dans *A. tumefaciens,* une origine de réplication fonctionnelle dans *A. tumefaciens,* les régions virB, virC et virG supplémentaires *d' A. tumefaciens* qui augmentent l'efficacité de transformation, les gènes de résistance à la tétracycline (Tétra) et à la spectinomycine (Spect) sous contrôle de promoteurs s'exprimant uniquement dans les bactéries,

On introduit dans ce vecteur un ADN de transfert (ADN-T) porteur de deux cassettes d'expression. La première cassette comprend : la séquence promotrice du CsVMV (WO 97/48819), une séquence issue de la séquence C4H du maïs (SEQ ID N° 1) en orientation antisens et la séquence terminateur NOS (nopaline synthase). La seconde cassette comprend : la séquence promotrice du gène d'actine de riz et la séquence du premier intron de ce gène, un gène de sélection à un herbicide, et une séquence terminateur NOS.

La transformation est effectuée via le protocole de transformation par A. *tumefaciens* d'Ishida et al. (Nature Biotechnology, 14, 745-750, 1996).

Des épis immatures d'une lignée produite en serre, sont prélevés 10 jours après la pollinisation et stérilisés pendant 15 minutes. Les embryons sont prélevés et mis en contact 5 minutes avec une suspension d'*Agrobacterium* contenant le vecteur décrit ci-dessus. Après avoir été retirés de la suspension d'*Agrobacterium*, les embryons sont mis en culture sur un milieu ne contenant ni bactériostatique, ni agent sélectif. Cette co-culture a lieu durant 4 à 7 jours à l'obscurité. Après la co-culture, les embryons sont repiqués sur un milieu de callogénèse frais contenant le bactériostatique et l'agent sélectif. Un cal s'initie et se développe à partir des cellules transformées de ces embryons. L'étape de callogénèse se passe à 25 °C à l'obscurité et dure 5 semaines. Les embryons-cals sont repiqués sur milieu frais toutes les 2 à 3 semaines. Au terme de cette étape les cals sont repiqués sur un milieu de régénération pendant 5 semaines avec repiquage du cal sur milieu frais après 2 à 3 semaines. On obtient une régénération de plantules à partir des cals, qui sont mises en enracinement en tube, une fois qu'elles sont assez développées. Après 10-15 jours en tube, les plantules sont acclimatées en phytotron avant d'être transférées en serre. Les transformants sont ensuite cultivés et croisés avec du pollen d'une plante non transgénique pour produire la génération T1.

### b- Transformation par Agrobacterium tumefaciens de plantes de maïs par un construit RNAi pour le gène C4H.

Construction d'un vecteur comprenant un fragment de la séquence du gène C4H en orientation sens et antisens. Ce vecteur est construit en utilisant un fragment de 200 à 700 bp issu de la séquence C4H grâce au système Gateway (Invitrogen). Ce vecteur porte notamment une cassette d'expression constituée du promoteur CsVMV, du fragment de la séquence C4H en orientation antisens, du premier intron du gène de la tubuline de riz, du fragment de la séquence C4H en orientation sens et du terminateur NOS.

Ce vecteur est utilisé pour la transformation du maïs par la méthode d'Ishida décrite ci-dessus.

### c- Analyses phénotypiques des transformants anti-sens et RNAi

Le protocole d'analyse par NIRs selon l'exemple 2 est utilisé pour évaluer la digestibilité. On évalue la tolérance aux *Fusarium* selon le protocole d'inoculation artificielle par *Fusarium monoliforme,* décrit dans l'exemple 3.

### Exemple 5 : Recherche d'allèles nouveaux pour le gène C4H dans les ressources génétiques ou dans une collection de mutations induites par un agent chimique chez le maïs

On utilise une collection représentative appelée ci-après « core collection » présentant une variabilité moléculaire maximale en relation avec des critères d'origines à la fois géographiques et temporels, afin d'identifier de nouveaux allèles du gène C4H par la méthode dite d'EcoTilling décrite dans la littérature (Mejlhede et al., 2006, Plant Breeding, 125 : 461-467).

Simultanément au criblage de la « core collection », une recherche « d'allèles induits » est réalisé à partir d'une collection de maïs issue de la réunion de collections de maïs muté par des radiations ionisantes ou un agent chimique (EMS). Cette collection de maïs permet d'identifier des allèles non présents naturellement dans les variétés de maïs existantes naturellement. De plus, 5% des mutations découvertes sont des allèles nuls du gènes C4H, c'est-à-dire un allèle du gène C4H où la fonction est annulée (absence d'expression). Cette méthode dite de TILLING (Targeting Induced Local Lésions IN Genomes) est décrite dans la littérature (McCallum et al. 2000, Nature Biotechnology. 18 : 455-457).

Les allèles issus des approches de Tilling ou d'EcoTilling sont identifiés grâce à la technologie développée par Henikoff et Comaï (Plant J. 2006 Feb;45(4):684-94, Annu Rev Plant Biol. 2003;54:375-401). Pour cela, des amorces spécifiques du gène C4H sont développées. Le produit PCR correspondant à l'amplification d'une copie du gène C4H est ensuite comparé avec un même produit PCR issu d'un ADN issue d'une variété de référence. La méthode d'analyse des mutations est réalisée par identification d'hétéroduplex d'ADN créés par la renaturation des brins d'ADN provenant de la copie de la variété testée et la variété de référence. En cas de présence d'une mutation dans la variété testée, il s'ensuit une anomalie d'appareillement (ou « mismatch »). Cette anomalie est détectée puis coupée par une enzyme de type endonucléase reconnaissant cette structure particulière (par exemple l'enzyme Cell ou l'enzyme Endol). La reconnaissance du mismatch est ensuite détectée par électrophorèse. Par exemple sur gel d'agarose, sur séquenceur automatique en plaque (type LICOR), sur séquenceur capillaire sur ABI3100 ou sur ABI3730.

Chaque différence détectée constitue un « haplotype » particulier du gène C4H. L'ensemble des variétés présentant des haplotypes distincts (provenant soit d'un criblage des ressources génétiques soit de la découverte de mutations induites artificiellement) sont croisés avec une variété de référence afin de disposer d'une collection d'allèles de C4H dans un même fond génétique élite.

Des évaluations phénotypiques sont entreprises pour déterminer si la présence d'un haplotype particulier est corrélée au caractère digestible ou à la résistance à la fusariose.

### Exemple 6 : Utilisation des allèles identifiés en sélection variétale

Les haplotypes conférant le plus fort niveau de digestibilité et de résistance/tolérance à la fusariose sont ensuite utilisés en sélection variétale. Le brassage génétique entre la variété résistante/tolérante et des variétés présentant d'autres qualités agronomiques telles que des rendements élevés, de fort taux en protéines, une aptitude à la résistance à la germination sur pied, etc... permet de sélectionner de nouvelles variétés regroupant l'ensemble des caractères souhaités. L'allèle conférant la résistance à la fusariose est suivi par marquage moléculaire au cours de ce processus de sélection variétale.

Ainsi les allèles identifiés par Tilling, EcoTilling ou validés par test d'association, permettent de proposer des plans de croisements entre les individus les plus complémentaires sur la base de leurs allèles (ici un ou plusieurs allèles conférant une résistance à la fusariose) puis d'utiliser les connaissances accumulées pour proposer les croisements pertinents des parents potentiels afin de pyramider des gènes (accumuler différents haploytypes) devant aboutir à l'obtention de variétés améliorées.

## Revendications

1. Maïs présentant un allèle du gène C4H, appelé D1938 comprenant une insertion d'un transposon après le nucléotide 2852 de SEQ ID N°1, ledit allèle étant présent dans un échantillon représentatif de graines déposées au NCIMB sous le numéro NCIMB 41507.

2. Maïs selon la revendication 1, **caractérisé en ce qu'**il comprend également un allèle du gène CCR1, appelé Δ3318, comprenant une insertion d'un transposon dans le premier intron dudit gène, ledit allèle étant présent dans un échantillon représentatif de graines déposées au NCIMB sous le numéro NCIMB 41236, et étant décrit dans la demande WO 2006/035045.

3. Grain de maïs présentant un allèle du gène C4H, appelé D1938 comprenant une insertion d'un transposon dans le premier intron dudit gène, ledit allèle étant présent dans les graines déposées au NCIMB sous le numéro NCIMB 41507.

4. Grain de maïs selon la revendication 3, **caractérisé en ce qu'**il comprend également un allèle du gène CCR1, appelé Δ3318, comprenant une insertion d'un transposon dans le premier intron dudit gène, ledit allèle étant présent dans un échantillon représentatif de graines déposées au NCIMB sous le numéro NCIMB 41236 et étant décrit dans la demande WO 2006/035045.

5. Méthode de sélection d'un maïs possédant des caractéristiques de digestibilité améliorée et de tolérance aux pathogènes fongiques, comprenant l'étape d'effectuer un génotypage d'une population de maïs et de sélectionner, parmi cette population, les individus porteurs de l'allèle du gène C4H appelé D1938 comprenant une insertion d'un transposon après le nucléotide 2852 de SEQ ID N° 1, ledit allèle étant présent dans un échantillon représentatif de graines déposées au NCIMB sous le numéro NCIMB 41507.

6. Méthode selon la revendication 5, dans laquelle ledit maïs contient également l'allèle Δ3318 comprenant une insertion d'un transposon dans le premier intron du gène CCR1, ledit allèle étant présent dans un échantillon représentatif de graines déposées au NCIMB sous le numéro NCIMB 41236 et étant décrit dans la demande WO 2006/035045.

7. Utilisation d'un maïs selon la revendication 1 ou 2 pour la préparation d'une composition destinée à l'alimentation animale.

8. Méthode pour la préparation d'une composition destinée à l'alimentation animale comprenant l'ensilage d'un maïs selon la revendication 1 ou 2.

9. Méthode pour augmenter la tolérance à un pathogène fongique chez le maïs comprenant une étape consistant à altérer qualitativement et/ou quantitativement la synthèse de lignines, par l'inhibition totale ou partielle de l'expression du gène codant pour la cinnamate 4-hydroxylase (C4H), la tolérance audit pathogène fongique étant augmentée par rapport à un maïs non altéré, **caractérisé en ce que** ladite inhibition est obtenue par insertion d'un transposon après le nucléotide 2852 de SEQ ID N°1, ledit maïs ainsi obtenu présentant un allèle du gène C4H, appelé D1938, ledit allèle étant présent dans un échantillon représentatif de graines déposées au NCIMB sous le numéro NCIMB 41507.

10. Méthode selon la revendication 9, **caractérisée en ce que** ledit pathogène est *Fusarium monoliforme.*

## Patentansprüche

1. Mais, das ein Allel des C4H-Gens mit der Bezeichnung D1938 aufweist, welches eine Insertion eines Transposons nach dem Nukleotid 2852 von SEQ ID Nr. 1 umfasst, wobei das Allel in einer repräsentativen Stichprobe von Körnern, die bei der NCIMB unter der Nummer NCIMB 41507 hinterlegt wurden, vorliegt.

2. Mais nach Anspruch 1, **dadurch gekennzeichnet, dass** er außerdem ein Allel des CCR1-Gens mit der Bezeichnung Δ3318 umfasst, das eine Insertion eines Transposons in das erste Intron des Gens umfasst, wobei das Allel in einer repräsentativen Stichprobe von Körnern, die bei der NCIMB unter der Nummer NCIMB 41236 hinterlegt wurden, vorliegt und in der Anmeldung WO 2006/035045 beschrieben ist.

3. Maiskorn, das ein Allel des C4H-Gens mit der Bezeichnung D1938 aufweist, das eine Insertion eines Transposons in das erste Intron des Gens umfasst, wobei das Allel in Körnern, die bei der NCIMB unter der Nummer NCIMB 41507 hinterlegt wurden, vorliegt.

4. Maiskorn nach Anspruch 3, **dadurch gekennzeichnet, dass** er außerdem ein Allel des CCR1-Gens mit der Bezeichnung Δ3318 umfasst, das eine Insertion eines Transposons in das erste Intron des Gens umfasst, wobei das Allel in einer repräsentativen Stichprobe von Körnern, die bei der NCIMB unter der Nummer NCIMB 41236 hinterlegt wurden, vorliegt und in der Anmeldung WO 2006/035045 beschrieben ist.

5. Verfahren zur Selektion eines Maises, der Merkmale von verbesserter Verdaulichkeit und Toleranz gegenüber pilzlichen Pathogenen aufweist, umfassend den Schritt des Durchführens einer Genotypbestimmung einer Maispopulation und des Selektierens von Einzelpflanzen aus dieser Population, die das Allel des C4H-Gens mit der Bezeichnung D1938 tragen, das eine Insertion eines Transposons nach dem Nukleotid 2852 der SEQ ID Nr. 1 umfasst, wobei das Allel in einer repräsentativen Stichprobe von Körnern, die bei der NCIMB unter der Nummer NCIMB 41507 hinterlegt wurden, vorliegt.

6. Verfahren nach Anspruch 5, wobei der Mais außerdem das Allel Δ3318 enthält, das eine Insertion eines Transposons in das erste Intron des CCR1-Gens umfasst, wobei das Allel in einer repräsentativen Stichprobe von Körnern, die bei der NCIMB unter der Nummer NCIMB 41236 hinterlegt wurden, vorliegt und in der Anmeldung WO 2006/035045 beschrieben ist.

7. Verwendung eines Maises nach Anspruch 1 oder 2 zur Herstellung einer Zusammensetzung, die für die tierische Ernährung bestimmt ist.

8. Verfahren zur Herstellung einer Zusammensetzung, die für die tierische Ernährung bestimmt ist, umfassend das Silieren eines Maises nach Anspruch 1 oder 2.

9. Verfahren zum Erhöhen der Toleranz des Maises gegen ein pilzliches Pathogen, umfassend einen Schritt, der daraus besteht, dass man die Synthese von Ligninen durch vollständige oder teilweise Hemmung der Expression des Gens, das für die Cinnamat-4-Hydroxylase (C4H) codiert, qualitativ und/oder quantitativ verändert, wobei die Toleranz gegen das pilzliche Pathogen im Vergleich zu einem nicht veränderten Mais erhöht ist, **dadurch gekennzeichnet, dass** die Hemmung durch Insertion eines Transposons nach dem Nukleotid 2852 von SEQ ID Nr. 1 erzielt wird, wobei der so erhaltene Mais ein Allel eines C4H-Gens mit der Bezeichnung D1938 aufweist, wobei das Allel in einer repräsentativen Stichprobe von Körnern, die bei der NCIMB unter der Nummer NCIMB 41507 hinterlegt wurden, vorliegt.

10. Verfahren nach Anspruch 9, **dadurch gekennzeichnet, dass** es sich bei dem Pathogen um *Fusarium monoliforme* handelt.

## Claims

1. Maize having an allele of the gene C4H, referred to as D1938, comprising an insertion of a transposon after the nucleotide 2852 of SEQ ID N0:1, said allele being present in a representative sample of seeds deposited with NCIMB under the number NCIMB 41507.

2. Maize according to Claim 1, **characterized in that** it also comprises an allele of the gene CCR1, referred to as Δ3318, comprising an insertion of a transposon in the first intron of said gene, said allele being present in a representative sample of seeds deposited with NCIMB under the number NCIMB 41236 and being described in the patent application WO 2006/035045.

3. Maize seed having an allele of the gene C4H, referred to as D1938, comprising an insertion of a transposon in the first intron of said gene, said allele being present in seeds deposited with NCIMB under the number NCIMB 41507.

4. Maize seed according to Claim 3, **characterized in that** it also comprises an allele of the gene CCR1, referred to as Δ3318, comprising an insertion of a transposon in the first intron of said gene, said allele being present in a representative sample of seeds deposited with NCIMB under the number NCIMB 41236 and being described in the patent application WO 2006/035045.

5. Method for selecting a maize having characteristics of improved digestibility and of tolerance to fungal pathogens, comprising the step of carrying out genotyping of a population of maize and of selecting, from this population, the individuals bearing the allele of the gene C4H, referred to as D1938, comprising an insertion of a transposon after the nucleotide 2852 of SEQ ID NO:1, said allele being present in a representative sample of seeds deposited with NCIMB under the number NCIMB 41507.

6. Method according to Claim 5, in which said maize also contains the allele Δ3318, comprising an insertion of a transposon in the first intron of the gene CCR1, said allele being present in a representative sample of seeds deposited with NCIMB under the number NCIMB 41236 and being described in the patent application WO 2006/035045.

7. Use of a maize according to Claim 1 or 2 for the preparation of a composition intended for animal feed.

8. Method for the preparation of a composition intended for animal feed, comprising silaging of a maize according to Claim 1 or 2.

9. Method for increasing the tolerance to a fungal pathogen in maize, comprising a step consisting in qualitatively and/or quantitatively altering lignin synthesis by the total or partial inhibition of the expression of the gene encoding cinnamate-4-hydroxylase (C4H), the tolerance to said fungal pathogen being increased compared to an unaltered maize, **characterized in that** said inhibition is obtained by insertion of a transposon after the nucleotide 2852 of SEQ ID NO:1, said maize thus obtained having an allele of the gene C4H, referred to as D1938, said allele being present in a representative sample of seeds deposited with NCIMB under the number NCIMB 41507.

10. Method according to Claim 9, **characterized in that** said pathogen is *Fusarium monoliforme.*


### SEQUENCE LISTING

<110> BIOGEMMA
<120> Maïs présentant une bonne digestibilité et une résistance aux maladies
<130> BGM 44 - WO
<150> FR 0609295
   <151> 2006-10-24
<160> 5
<170> PatentIn version 3.3
<210> 1
   <211> 4254
   <212> DNA
   <213> Zea mays
<400> 1
   catgtgtagg aacttctaat tggcctctta aggatggaca tctttcatca atttattaga      60
   tttagtgaga ggctccaaga tggtatatgt agaatgggag gtaaagacaa actttggcca     120
   gtttacattt tcaaaactac aactatatat cttgcctatc cttgtttact ataaataaga     180
   gtggtaatgg atcacgatcc aaatgttttt cgtaatttat ttgtgccttt aattaatatt     240
   agttcaaaat tgaatagaat tagagtccaa tcttaatcga tctgacactt aaaattttta     300
   gtgtaaaatt tagagtcatt accaccccta actataagaa cccatcacag tccacttatc      360
   tgcacattct ctaattttgt tctgtgaatt agctgaagca tttttgtaag gacttggctg     420
   cgtttcgtaa ttagtaacag taaacttttc catggaaaag acgggttagc ggagctgcaa     480
   cttcaccgac tgcagtctca gcggtgatcc atgaatcccc tttgggcctt ccgctgccag     540
   caaatagcaa tagtagtgtt tgatcccgtt ccaaaaacaa aaacaaaaag aagagccaca     600
   cattttggaa acttttttgt ggggcccgcc cgcagcacca aaaccatacc aacccccgca      660
   atggcaaacc aaacaaattc gtgtactcaa attggacccg tctcggcgct ctctcccaaa     720
   aaaacgctat aaaatccccg gcattcctcc ttgcctgttc catcgcctcc acgaacaaac     780
   cacacacccc acctaccccg gccggaccgg caggcagcac agcatggacc tcgccctcct     840
   agagaaggcc ctgctgggcc tgttcgccgc ggctgtggtg gccatcgccg tggccaagct     900
   gaccggcaag cggtaccgcc tcccaccggg gcccccgggc gcccccgtgg tgggaaactg     960
   gctgcaggtg ggcgacgacc tgaaccaccg caacctgatg gccatggcga agcggttcgg    1020
   cgacatcttc ctgctgcgca tgggcgtgcg caacctggtg gtggtgtcga ccccggagct    1080
   ggccaaggag gtgctccaca cgcagggcgt ggagttcggc tcccgcaccc gcaacgtggt    1140
   gttcgacatc ttcacgggca aggggcagga catggtgttc acggtgtacg gcgaccactg    1200
   gcgcaagatg cggcgcatca tgaccgtccc cttcttcacc aacaaggtgg tggcccagaa 1260
   ccgcgccggg tgggaggagg aggcccggct ggtggtggag gacgtgagga aggaccccga 1320
   ggccgcggcc ggcggcgtcg tgctccgccg ccgcctccag ctgatgatgt acaacgacat 1380
   gttccgcatc atgttcgacc gccggttcga cagcgagcac gacccgctct tcaacaagct 1440
   caaggcgctc aacgcggagc gcagccgcct gtcgcagagc ttcgagtaca actacggcga 1500
   cttcatcccc gtgctccgcc ccttcctccg cggctacctc aaccgctgcc acgacctcaa 1560
   gacgcgccgc atgaaggtct tcgaggacaa cttcgtacag gagcgcaagt aagtctgcca 1620
   tattccattt tctttccttt cctttttctt ttgagctggc cacctgggct ggtggttggt 1680
   tgacggcggg tggagttgca agttgcatcg tcggcgggtg aggtatgagc tggaggctgg 1740
   agctgggcgg aagacagttg catgcattgc atctgtgcct ctgtggtggt ggctgcgcac 1800
   atgctgcaac cgatgccatc taccggtgga caaatcaatg ccatctgcca gtggacaagg 1860
   acgaatcaac agccaagtcc atattttatt ttattttgct agtcattgaa ttttggcgag 1920
   atcggacgac ttttgtgatg catggctggc tccagtcatg tttgacacgt aactaaaaga 1980
   cccaccgcac gggttaggga gtactagtac ctactactcc taaacaacaa cttgtagttg 2040
   tccacaacag gttaacagat caaagaagga taggtgagcg gcactactca tctcgtggct 2100
   tccggtgaca gatgatcagc ccacgaagga aaaagaaaaa gggactcgct tgatttgcgt 2160
   tgctttgctt tccgcgcaag gccgaaagct taaggtcggg gcaacgacac accgagagcg 2220
   acggagatat accacgctct gctgcttcat gatagctgga aggacatcag catgacacgc 2280
   atgccaataa tgtagtagaa gactagttta tttgtcacct actaggcaga gagtgcggtg 2340
   gttccatgaa ccaaccattc gtgcgtgcgt gcccaccgca cgaccacgag ggggcatgca 2400
   acatatactt tacaaaattc attcggttgt tgacttggtt tcaattcatt gggttgggaa 2460
   gacaaaacta acatggatgc gttgcgtact cacaggaagg tgatggctca gactggtgag 2520
   atccggtgcg ccatggatca catcctcgag gccgagagga agggcgagat caaccacgac 2580
   aacgtcctct acatcgtcga gaacatcaac gtcgcaggta tcgataatcg tcactgctca 2640
   gtgcaatgaa acggaaacag agtatgctat gtctgaagaa ccaagtgaga cttatcattc 2700
   aactactgca gtccagcgct actacacaaa caaatagcac tgcaatatcg gaagatataa 2760
   taatataagg cacgtcttaa tcaagtctcc gtcagccaac ttaattccgt ccttaattaa 2820
   ccggcaacat actgagccta atgcggtgtc acacttcaag tgggctcccg tatttatctg 2880
   ctaggcatag tatctatctg tgtatcaaaa ggtagttgaa gccgccacca cgtgaccacg 2940
   cctcaaaaag aattgcgttt tgatttgtat atccaagctg ccacacctgc aggcagtcgg 3000
   tggcaaaaaa aaaaaactaa tttgtttgct tttgcagcga tcgagacgac gctgtggtcg 3060
   atcgagtggg gcatcgccga gctggtgaac cacccggcca tccagcacaa gctccgggag 3120
   gagctcgcct cggtgctggg cgccggcgtg cctgtgacgg agccggacct cgagcgcctc 3180
   ccctaccttc aggccatcgt caaggagacg ctccgcctgc gcatggccat cccgctgctg 3240
   gtcccccaca tgaacctcaa cgacggcaag ctcgccggct tcgacatccc cgccgagtcc 3300
   aagatcctcg tcaatgcctg gttcctcgcc aacgacccca agaggtgggt gcgccccgac 3360
   gagttccggc ccgagcgctt cctggaggag gagaagtccg tggaggccca cggcaacgac 3420
   ttccgattcg tgccctttgg ggtcggccgc cggagctgcc ctgggatcat cctcgcgctg 3480
   cctatcatcg gcatcaccct gggccggctg gtgcagaact tccagctgct gccgccgccg 3540
   gggctggaca agatcgacac cacggagaag cccggccagt tcagcaacca gatcgccaag 3600
   catgccacca tcgtctgcaa gcccctcgag gcctagaaat caatgcgtgt ttcctgcacg 3660
   cgcccccgca gatgaagcac tatgtatttt ctcttttttt tgtgtgttgt gtttttttta 3720
   ctaagaggag atgtatttct tgttcgtaaa atgcacttag tcaaatggat cgagattatg 3780
   ttgatcatta aacaccaaaa tcacttagtc aaacgggtcg ggatccattt tcctcacaat 3840
   gtcaaccttt gactgatgac aatataatca aagcaagcaa atattaaata aaaaaatatg 3900
   tactctactt gcaatagatg catgtgcata tgcaacgtgt atgagaaact aaattgatag 3960
   atatgcggtc atttagagca tataggaaga catgttaaag tatacacgaa gctatcaaat 4020
   acttgccctc taaaatattt ctctccctaa aagaaagttc tccacatttg gaaaaacagt 4080
   ctctcccttt gaggaaaagg acatacaccc cctattggaa aactctcttt caatacaaga 4140
   ggaaaaaaag acaagaatga atagactaac tccccttaat ttatgcatag ttaaatggta 4200
   aaggatattc tagtgatacc aaataaagaa tttaaaagca tatgcatatg tagg 4254
<210> 2
   <211> 1669
   <212> DNA
   <213> Zea mays
<400> 2
<210> 3
   <211> 21
   <212> DNA
   <213> Artificial
<220>
   <223> amorce C4HH14intsp7
<400> 3
   cacgtcttaa tcaagtctcc g 21
<210> 4
   <211> 22
   <212> DNA
   <213> Artificial
<220>
   <223> amorce C4H-3_comp
<400> 4
   gttcatgtgg gggaccagca gc 22
<210> 5
   <211> 25
   <212> DNA
   <213> artificial
<220>
   <223> amorce OMuA
<400> 5
   cttcgtccat aatggcaatt atctc 25